# EUROPÄISCHE PATENTSCHRIFT

(11) **EP 3 433 350 B1**
(45) Veröffentlichungstag und Bekanntmachung des Hinweises auf die Patenterteilung: **13.10.2021**
(21) Anmeldenummer: 17709664.1
(22) Anmeldetag: 09.03.2017
(51) Int. Cl.: C11D 3/386, C12N 9/20

(54) **LIPASEN FÜR DEN EINSATZ IN WASCH- UND REINIGUNGSMITTELN**
LIPASES FOR USE IN WASHING- AND CLEANINGAGENTS
LIPASES UTILISE DANS DES AGENTS DE LAVAGE ET DE NETTOYAGE

(30) Priorität: 23.03.2016 DE 102016204813
(43) Veröffentlichungstag der Anmeldung: 30.01.2019
(73) Patentinhaber: Henkel AG & Co. KGaA, 40589 Düsseldorf (DE)
(72) Erfinder: HERBST, Daniela, 40237 Düsseldorf (DE); O'CONNELL, Timothy, 86899 Landsberg am Lech (DE); MUSSMANN, Nina, 47877 Willich (DE); EICHSTÄDT, Renée Charlott, 50733 Köln (DE); LINDNER, Claudia, 42719 Solingen (DE); KÖRFER, Georgette, 52224 Stolberg (DE); DAVARI DOLATABADI, Mehdi, 52072 Aachen (DE); VOJCIC, Ljubica, 95134 San Jose (US); SCHWANEBERG, Ulrich, 4728 Kelmis-Hergenrath (BE)
(86) Internationale Anmeldenummer: PCT/EP2017/055500
(87) Internationale Veröffentlichungsnummer: WO 2017/162440

(56) Entgegenhaltungen:
- WO-A1-2007/087503
- WO-A2-2006/084470
- WO-A2-2007/087244
- US-A1- 2007 179 074

### SEQUENCE LISTING

<110> Henkel AG & Co. KGaA
<120> Lipasen für den Einsatz in Wasch- und Reinigungsmitteln
<130> PT033756PCT
<150> 102016204813.9
   <151> 2016-03-23
<160> 5
<170> PatentIn version 3.5
<210> 1
   <211> 269
   <212> PRT
   <213> Thermomyces lanuginosus
<400> 1
<210> 2
   <211> 269
   <212> PRT
   <213> Artificial
<220>
   <223> Optimized variant of SEQ ID NO:1
<400> 2
   Glu Val Ser Gln Asp Leu Phe Asn Gln Phe Asn Leu Phe Ala Gln Tyr
   1               5                   10                  15
   Ser Ala Ala Ala Tyr Cys Gly Lys Asn Asn Asp Ala Pro Ala Gly Thr
               20                  25                  30
   Asn Ile Thr Cys Thr Gly Asn Ala Cys Pro Glu Val Glu Lys Ala Asp
           35                  40                  45
   Ala Thr Phe Leu Tyr Ser Phe Glu Asp Ser Gly Val Gly Asp Val Thr
       50                  55                  60
   Gly Phe Leu Ala Leu Asp Asn Thr Asn Lys Leu Ile Val Leu Ser Phe
   65                  70                  75                  80
   Arg Gly Ser Arg Ser Ile Glu Asn Trp Ile Gly Asn Leu Asn Phe Asp
                   85                  90                  95
   Leu Lys Glu Ile Asn Asp Ile Cys Ser Gly Cys Arg Gly His Asp Gly
               100                 105                 110
   Phe Thr Ser Ser Trp Arg Ser Val Ala Asp Thr Leu Arg Gln Lys Val
   115 120 125
   Glu Asp Ala Val Arg Glu His Pro Asp Tyr Arg Val Val Phe Thr Gly
   130 135 140
   His Ser Leu Gly Gly Ala Leu Ala Thr Val Ala Gly Ala Asp Leu Arg
   145 150 155 160
   Gly Asn Gly Tyr Asp Ile Asp Val Phe Ser Tyr Gly Ala Pro Arg Val
   165 170 175
   Gly Asn Arg Ala Phe Ala Glu Phe Leu Thr Val Gln Thr Gly Gly Thr
   180 185 190
   Leu Tyr Arg Ile Thr His Thr Asn Asp Ile Val Pro Arg Leu Pro Pro
   195 200 205
   Arg Glu Phe Gly Tyr Ser His Ser Ser Pro Glu Tyr Trp Ile Lys Ser
   210 215 220
   Gly Thr Leu Val Pro Val Arg Arg Arg Asp Ile Val Lys Ile Glu Gly
   225 230 235 240
   Ile Asp Ala Thr Gly Gly Asn Asn Gln Pro Asn Ile Pro Asp Ile Pro
   245 250 255
   Ala His Leu Trp Tyr Phe Gly Leu Ile Gly Thr Cys Leu
   260 265
<210> 3
   <211> 269
   <212> PRT
   <213> Artificial
<220>
   <223> Optimized variant of SEQ ID NO:1
<400> 3
   Glu Val Ser Gln Asp Leu Phe Asn Gln Phe Asn Leu Phe Ala Gln Tyr
   1 5 10 15
   Ser Ala Ala Ala Tyr Cys Gly Lys Asn Asn Asp Ala Pro Ala Gly Thr
   20 25 30
   Asn Ile Thr Cys Thr Gly Asn Ala Cys Pro Glu Val Glu Lys Ala Asp
   35 40 45
   Ala Thr Phe Leu Tyr Ser Phe Glu Asp Ser Gly Val Gly Asp Val Thr
       50                  55                  60
   Gly Phe Leu Ala Leu Asp Asn Thr Asn Lys Leu Ile Val Leu Ser Phe
   65                  70                  75                  80
   Arg Gly Ser Arg Ser Ile Glu Asn Trp Ile Gly Asn Leu Asn Phe Asp
                   85                  90                  95
   Leu Lys Glu Ile Asn Asp Ile Cys Ser Gly Cys Arg Gly His Asp Gly
               100                 105                 110
   Phe Thr Ser Ser Trp Arg Ser Val Ala Asp Thr Leu Arg Gln Lys Val
           115                 120                 125
   Glu Asp Ala Val Arg Glu His Pro Asp Tyr Arg Val Val Phe Thr Gly
       130                 135                 140
   His Ser Leu Gly Gly Ala Leu Ala Thr Val Ala Gly Ala Asp Leu Arg
   145                 150                 155                 160
   Gly Asn Gly Tyr Asp Ile Asp Val Phe Ser Tyr Gly Ala Pro Arg Val
                   165                 170                 175
   Gly Asn Arg Ala Phe Ala Glu Phe Leu Thr Val Gln Thr Gly Gly Thr
               180                 185                 190
   Leu Tyr Arg Ile Thr His Thr Asn Asp Ile Val Pro Arg Leu Pro Asn
           195                 200                 205
   Arg Glu Phe Gly Tyr Ser His Ser Ser Pro Glu Tyr Trp Ile Lys Ser
       210                 215                 220
   Gly Thr Leu Val Pro Val Arg Arg Arg Asp Ile Val Lys Ile Glu Gly
   225                 230                 235                 240
   Ile Asp Ala Thr Gly Gly Asn Asn Gln Pro Asn Ile Pro Asp Ile Pro
                   245                 250                 255
   Ala His Leu Trp Tyr Phe Gly Leu Ile Gly Thr Cys Leu
               260                 265
<210> 4
   <211> 269
   <212> PRT
   <213> Artificial
<220>
   <223> Optimized variant of SEQ ID NO:1
<400> 4
   Glu Val Ser Gln Asp Leu Phe Asn Gln Phe Asn Leu Phe Ala Gln Tyr
   1               5                   10                  15
   Ser Ala Ala Ala Tyr Cys Gly Lys Asn Asn Asp Ala Pro Ala Gly Thr
               20                  25                  30
   Asn Ile Thr Cys Thr Gly Asn Ala Cys Pro Glu Val Glu Lys Ala Asp
           35                  40                  45
   Ala Thr Phe Leu Tyr Ser Phe Glu Asp Ser Gly Val Gly Asp Val Thr
       50                  55                  60
   Gly Phe Leu Ala Leu Asp Asn Thr Asn Lys Leu Ile Val Leu Ser Phe
   65                  70                  75                  80
   Arg Gly Ser Arg Ser Ile Glu Asn Trp Ile Gly Asn Leu Asn Phe Asp
                   85                  90                  95
   Leu Lys Glu Ile Asn Asp Ile Cys Ser Gly Cys Arg Gly His Asp Gly
               100                 105                 110
   Phe Thr Ser Ser Trp Arg Ser Val Ala Asp Thr Leu Arg Gln Lys Val
           115                 120                 125
   Glu Asp Ala Val Arg Glu His Pro Asp Tyr Arg Val Val Phe Thr Gly
       130                 135                 140
   His Ser Leu Gly Gly Ala Leu Ala Thr Val Ala Gly Ala Asp Leu Arg
   145                 150                 155                 160
   Gly Asn Gly Tyr Asp Ile Asp Val Phe Ser Tyr Gly Ala Pro Arg Val
                   165                 170                 175
   Gly Asn Arg Ala Phe Ala Glu Phe Leu Thr Val Gln Thr Gly Gly Thr
               180                 185                 190
   Leu Tyr Arg Ile Thr His Thr Asn Asp Ile Val Pro Arg Leu Pro Pro
           195                 200                 205
   Arg Glu Asn Gly Tyr Ser His Ser Ser Pro Glu Tyr Trp Ile Lys Ser
       210                 215                 220
   Gly Thr Leu Val Pro Val Arg Arg Arg Asp Ile Val Lys Ile Glu Gly
   225                 230                 235                 240
   Ile Asp Ala Thr Gly Gly Asn Asn Gln Pro Asn Ile Pro Asp Ile Pro
                   245                 250                 255
   Ala His Leu Trp Tyr Phe Gly Leu Ile Gly Thr Cys Leu
               260                 265
<210> 5
   <211> 269
   <212> PRT
   <213> Artificial
<220>
   <223> Optimized variant of SEQ ID NO:1
<400> 5
   Glu Val Ser Gln Asp Leu Phe Asn Gln Phe Asn Leu Phe Ala Gln Tyr
   1               5                   10                  15
   Ser Ala Ala Ala Tyr Cys Gly Lys Asn Asn Asp Ala Pro Ala Gly Thr
               20                  25                  30
   Asn Ile Thr Cys Thr Gly Asn Ala Cys Pro Glu Val Glu Lys Ala Asp
           35                  40                  45
   Ala Thr Phe Leu Tyr Ser Phe Glu Asp Ser Gly Val Gly Asp Val Thr
       50                  55                  60
   Gly Phe Leu Ala Leu Asp Asn Thr Asn Lys Leu Ile Val Leu Ser Phe
   65                  70                  75                  80
   Arg Gly Ser Arg Ser Ile Glu Asn Trp Ile Gly Asn Leu Asn Phe Asp
                   85                  90                  95
   Leu Lys Glu Ile Asn Asp Ile Cys Ser Gly Cys Arg Gly His Asp Gly
               100                 105                 110
   Phe Thr Ser Ser Trp Arg Ser Val Ala Asp Thr Leu Arg Gln Lys Val
           115                 120                 125
   Glu Asp Ala Val Arg Glu His Pro Asp Tyr Arg Val Val Phe Thr Gly
       130                 135                 140
   His Ser Leu Gly Gly Ala Leu Ala Thr Val Ala Gly Ala Asp Leu Arg
   145                 150                 155                 160
   Gly Asn Gly Tyr Asp Ile Asp Val Phe Ser Tyr Gly Ala Pro Arg Val
                   165                 170                 175
   Gly Asn Arg Ala Phe Ala Glu Phe Leu Thr Val Gln Thr Gly Gly Thr
               180                 185                 190
   Leu Tyr Arg Ile Thr His Thr Asn Asp Ile Val Pro Arg Leu Pro Pro
           195                 200                 205
   Arg Glu Phe Gly Tyr Ser His Ser Ser Pro Glu Tyr Trp Ile Lys Ser
       210                 215                 220
   Gly Thr Leu Val Pro Val Arg Arg Arg Asp Ile Val Lys Ile Glu Gly
   225                 230                 235                 240
   Ile Asp Ala Thr Gly Gly Asn Asn Gln Pro Asn Ile Asn Asp Ile Pro
                   245                 250                 255
   Ala His Leu Trp Tyr Phe Gly Leu Ile Gly Thr Cys Leu
               260                 265

## Beschreibung

Die Erfindung liegt auf dem Gebiet der Enzymtechnologie. Die Erfindung betrifft insbesondere Lipasen sowie deren Herstellung, deren Aminosäuresequenz, die insbesondere im Hinblick auf den Einsatz in Wasch- und Reinigungsmitteln verändert wurden, alle hinreichend ähnlichen Lipasen mit einer entsprechenden Veränderung und für sie codierende Nukleinsäuren. Die Erfindung betrifft ferner Verfahren und Verwendungen dieser Lipasen sowie sie enthaltende Mittel, insbesondere Wasch- und Reinigungsmittel.

Lipasen gehören zu den technisch bedeutendsten Enzymen überhaupt. Ihr Einsatz für Wasch- und Reinigungsmittel ist industriell etabliert und sie sind in praktisch allen modernen, leistungsfähigen Wasch- und Reinigungsmitteln enthalten. Eine Lipase ist ein Enzym, das die Hydrolyse von Esterbindungen in Lipid-Substraten, insbesondere in Fetten und Ölen, katalysiert. Lipasen stellen daher eine Gruppe der Esterasen dar. Lipasen sind im Allgemeinen vielseitige Enzyme, die eine Vielzahl an Substraten akzeptieren, beispielsweise aliphatische, alizyklische, bizyklische und aromatische Ester, Thioester und aktivierte Amine. Lipasen wirken gegen Fettrückstände in der Wäsche und katalysieren deren Hydrolyse (Lipolyse). Lipasen mit breiten Substratspektren werden insbesondere dort verwendet, wo inhomogene Rohstoffe oder Substratgemische umgesetzt werden müssen, also beispielsweise in Wasch- und Reinigungsmitteln, da Verschmutzungen aus unterschiedlich aufgebauten Fetten und Ölen bestehen können. Die in den aus dem Stand der Technik bekannten Wasch- oder Reinigungsmitteln eingesetzten Lipasen sind üblicherweise mikrobiellen Ursprungs und stammen in der Regel aus Bakterien oder Pilzen, beispielsweise der Gattungen *Thermomyces, Bacillus, Pseudomonas, Acinetobacter, Micrococcus, Humicola, Trichoderma* oder *Trichosporon.* Lipasen werden üblicherweise nach an sich bekannten biotechnologischen Verfahren durch geeignete Mikroorganismen produziert, beispielsweise durch transgene Expressionswirte der Gattungen Bacillus oder durch filamentöse Pilze.

In der deutschen Patentanmeldung DE 102012224038 A1 und der internationalen Patentanmeldung WO 2014/152674 ist beispielsweise eine für Wasch- und Reinigungsmittel vorgesehene Lipase aus *Thermomyces lanuginosus* (SEQ ID NO:2) offenbart. Weitere Lipasen für den Einsatz in Wasch- und Reinigungsmittel sind aus US 2007/0179074 A1, WO 2007/087244 A2, WO2007/087503 A1 und WO 2006/084470 A2 bekannt. Generell sind nur ausgewählte Lipasen für den Einsatz in flüssigen Tensidzubereitungen überhaupt geeignet. Viele Lipasen zeigen in derartigen Zubereitungen keine ausreichende katalytische Leistung oder Stabilität. Allerdings entsteht durch die Verwendung von Lipasen nach dem Waschvorgang ein unangenehmer Geruch auf den gewaschenen Objekten, insbesondere Textilien, da die Lipase durch die Textilien absorbiert wird und weiterhin Fettsäureester zu Fettsäuren mit niedrigem Molekulargewicht hydrolysiert. Diese niedrig molekularen Fettsäuren werden dann durch ihren unangenehmen Geruch wahrgenommen. Die Intensität des Geruchs kann aufgrund der Beschaffenheit des Textils variieren. Daher ist es wünschenswert für jede Art von Textilien eine Lipase zu finden, die eine ausreichende Waschleistung zeigt und gleichzeitig eine minimale Geruchsemission hervorruft.

Die aus dem Stand der Technik bekannten Lipasen erzeugen folglich eine Geruchsemission auf gewaschenen Textilien. Insbesondere sind diese Lipasen auch nicht auf eine Minimierung ihrer Geruchsemission auf verschiedenen Textilien optimiert sind.

Überraschenderweise wurde jetzt festgestellt, dass eine Lipase aus *Thermomyces lanuginosus* oder eine hierzu hinreichend ähnliche Lipase (bezogen auf die Sequenzidentität), die mindestens eine Aminosäuresubstitution an Position 83, 86, 87, 89, 90, 92, 93, 95, 113, 174, 202, 203, 205, 206, 207, 208, 209, 211, 227, 252, 253, 256, 258, 259, 260, 264, oder 267, jeweils bezogen auf die Nummerierung gemäß SEQ ID NO:1, aufweist, besonders für den Einsatz in Wasch- oder Reinigungsmitteln geeignet und eine verminderte Geruchsemission, insbesondere auf Baumwoll- und Elasthan-haltigen Textilien, hervorruft.

Gegenstand der Erfindung ist daher in einem ersten Aspekt eine Lipase umfassend eine Aminosäuresequenz, die mindestens 70 % Sequenzidentität mit der in SEQ ID NO:1 angegebenen Aminosäuresequenz über deren Gesamtlänge aufweist und die mindestens eine Aminosäuresubstitution an Position 83, 86, 87, 89, 90, 92, 93, 95, 113, 174, 202, 203, 205, 206, 207, 208, 209, 211, 227, 252, 253, 256, 258, 259, 260, 264, oder 267, jeweils bezogen auf die Nummerierung gemäß SEQ ID NO:1, aufweist, wobei die mindestens eine Aminosäuresubstitution aus der aus 208N und 253N bestehenden Gruppe ausgewählt ist.

Ein weiterer Gegenstand der Erfindung ist ein Verfahren zur Herstellung einer solchen Lipase umfassend das Substituieren mindestens einer Aminosäure an mindestens einer Position, die den Positionen 83, 86, 87, 89, 90, 92, 93, 95, 113, 174, 202, 203, 205, 206, 207, 208, 209, 211, 227, 252, 253, 256, 258, 259, 260, 264, oder 267 in SEQ ID NO:1 entspricht, in einer Ausgangslipase, die mindestens 70 % Sequenzidentität zu der in SEQ ID NO:1 angegebenen Aminosäuresequenz über deren Gesamtlänge aufweist, vorzugsweise derart, dass die Lipase an mindestens einer Position mindestens eine der Aminosäuresubstitutionen P208N, P253N umfasst Eine Lipase im Sinne der vorliegenden Patentanmeldung umfasst daher sowohl die Lipase als solche als auch eine mit einem erfindungsgemäßen Verfahren hergestellte Lipase. Alle Ausführungen zur Lipase beziehen sich daher sowohl auf die Lipase als Stoff wie auch auf die entsprechenden Verfahren, insbesondere Herstellungsverfahren der Lipase.

Als weitere Erfindungsgegenstände sind mit den erfindungsgemäßen Lipasen beziehungsweise den Herstellungsverfahren für erfindungsgemäße Lipasen für diese Lipasen codierende Nukleinsäuren, erfindungsgemäße Lipasen oder Nukleinsäuren enthaltende nicht menschliche Wirtszellen sowie erfindungsgemäße Lipasen umfassende Mittel, insbesondere Wasch- und Reinigungsmittel, Wasch- und Reinigungsverfahren, und über erfindungsgemäßen Lipasen definierte Verwendungen verbunden.

Die vorliegende Erfindung basiert auf der überraschenden Erkenntnis der Erfinder, dass eine erfindungsgemäße Veränderung an mindestens einer der Positionen 83, 86, 87, 89, 90, 92, 93, 95, 113,174, 202, 203, 205, 206, 207, 208, 209, 211, 227, 252, 253, 256, 258, 259, 260, 264, oder 267 der Lipase aus *Thermomyces lanuginosus* gemäß SEQ ID NO:1, in einer Lipase, die eine zu der in SEQ ID NO:1 angegebenen Aminosäuresequenz zu mindestens 70% identische Aminosäuresequenz umfasst, derart, dass an mindestens einer der entsprechenden Positionen eine andere Aminosäure vorhanden sind, eine minimierte Geruchsemission, insbesondere auf Baumwoll- und Elasthan-haltigen Textilien, bewirkt. Das ist insbesondere insoweit überraschend, als dass keine der oben genannten Aminosäuresubstitutionen zuvor mit einer verminderten Geruchsemission der Lipase in Verbindung gebracht wurde. Besonders vorteilhafte Effekte wurden für solche Lipasen gefunden, die Substitutionen an den Positionen 208, 211 oder 253, insbesondere 208N, 211N oder 253N, aufweisen.

Die erfindungsgemäßen Lipasen bewirken eine verminderte Geruchsemission auf Textilien nach dem Waschvorgang, beispielsweise nach dem Waschen von Baumwoll- und Elasthan-haltigen Textilien. Dieser Vorteil ist unabhängig gegenüber den verwendeten Tensiden und/oder Bleichmitteln, den verwendeten Waschtemperaturen, sauren oder alkalischen Bedingungen, den verwendeten pH-Wert-Bedingungen, denaturierenden oder oxidierenden Agentien und gegenüber einer Veränderung der Redox-Verhältnisse. Mit besonders bevorzugten Ausführungsformen der Erfindung werden folglich leistungsverbesserte Lipase-Varianten bereitgestellt. Solche vorteilhaften Ausführungsformen erfindungsgemäßer Lipasen ermöglichen folglich verbesserte Waschergebnisse an lipolytisch-sensitiven Anschmutzungen und eine herabgesetzte Geruchsemission nach dem Waschvorgang.

Hinsichtlich der einleitend erwähnten deutschen Patentanmeldung DE 102012224038 A1 und der internationalen Patentanmeldung WO 2014/152674 handelt es bei der vorliegenden Erfindung daher um eine alternative Sequenzveränderung, die zur Reduzierung der Geruchsemission nach dem Waschen führt. Das ist insofern überraschend, als dass die Substitution mindestens einer Aminosäure an Position 83, 86, 87, 89, 90, 92, 93, 95, 113, 174, 202, 203, 205, 206, 207, 208, 209, 211, 227, 252, 253, 256, 258, 259, 260, 264, oder 267, jeweils bezogen auf die Nummerierung gemäß SEQ ID NO:1, zuvor nicht mit einer verminderten Geruchsemission der Lipase in Verbindung gebracht wurde.

Eine erfindungsgemäße Lipase weist eine enzymatische Aktivität auf, das heißt, sie ist zur Hydrolyse von Fetten und Ölen befähigt, insbesondere in einem Wasch- oder Reinigungsmittel. Eine erfindungsgemäße Lipase ist daher ein Enzym, welches die Hydrolyse von Esterbindungen in Lipid-Substraten katalysiert und dadurch in der Lage ist, Fette oder Öle zu spalten. Ferner handelt es sich bei einer erfindungsgemäßen Lipase vorzugsweise um eine reife (mature) Lipase, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen enthält die erfindungsgemäße Lipase mindestens eine Aminosäuresubstitution, die aus der Gruppe bestehend aus P208N und P253N, jeweils bezogen auf die Nummerierung gemäß SEQ ID NO:1, ausgewählt ist. In anderen bevorzugten Ausführungsformen enthält die erfindungsgemäße Lipase mindestens zwei oder drei der Aminosäuresubstitutionen P208N, F211N, und P253N, wobei die Nummerierung jeweils bezogen ist auf die Nummerierung gemäß SEQ ID NO:1.

In verschiedenen Ausführungsformen umfasst die erfindungsgemäße Lipase ferner die Aminosäuresubstitutionen T231R und N233R, jeweils bezogen auf die Nummerierung gemäß SEQ ID NO:1. Diese Substitutionen sind im Stand der Technik bekannt und sind beispielsweise in der bereits kommerziell erhältlichen Lipase Lipex Evity 100 T enthalten.

In einer weiteren Ausführungsform der Erfindung umfasst die Lipase eine Aminosäuresequenz, die zu der in SEQ ID NO:1 angegebenen Aminosäuresequenz über deren Gesamtlänge zu mindestens 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die an mindestens einer der Positionen, die den Positionen 83, 86, 87, 89, 90, 92, 93, 95, 113, 174, 202, 203, 205, 206, 207, 208, 209, 211, 227, 252, 253, 256, 258, 259, 260, 264, oder 267 in der Zählung gemäß SEQ ID NO:1 entsprechen, eine Aminosäuresubstitution aufweist, wobei die mindestens eine Aminosäuresubstitution aus der aus 208N und 253N bestehenden Gruppe ausgewählt ist. Im Zusammenhang mit der vorliegenden Erfindung bedeutet das Merkmal, dass eine Lipase die angegebenen Substitutionen aufweist, dass sie mindestens eine nicht-native Aminosäure an einer der entsprechenden Positionen enthält, vorzugsweise eine der explizit genannten, d.h. nicht alle der genannten Positionen anderweitig mutiert oder, beispielsweise durch Fragmentierung der Lipase, deletiert sind. Derartige Lipasen, die erfindungsgemäß bevorzugt sind, sind in SEQ ID Nos:3-5 angegeben.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure-oderAminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

In einer weiteren Ausführungsform der Erfindung ist die Lipase dadurch gekennzeichnet, dass ihre Reinigungsleistung gegenüber derjenigen einer Lipase, die eine Aminosäuresequenz umfasst, die einer der in SEQ ID Nos: 1-5 angegebenen Aminosäuresequenzen entspricht, nicht signifikant verringert ist, d.h. mindestens 80 % der Referenzwaschleistung besitzt. Die Reinigungsleistung kann in einem Waschsystem bestimmt werden, das ein Waschmittel in einer Dosierung zwischen 4,5 und 7,0 Gramm pro Liter Waschflotte sowie die Lipase enthält, wobei die zu vergleichenden Lipasen konzentrationsgleich (bezogen auf aktives Protein) eingesetzt sind und die Reinigungsleistung gegenüber einer Anschmutzung auf Baumwolle bestimmt wird durch Messung des Reinigungsgrades der gewaschenen Textilien. Beispielsweise kann der Waschvorgang für 70 Minuten bei einer Temperatur von 40°C erfolgen und das Wasser eine Wasserhärte zwischen 15,5 und 16,5° (deutsche Härte) aufweisen. Die Konzentration der Lipase in dem für dieses Waschsystem bestimmten Waschmittel beträgt von 0,001-0,1 Gew.-%, vorzugsweise von 0,01 bis 0,06 Gew.-%, bezogen auf aktives, gereinigtes Protein.

Ein bevorzugtes flüssiges Waschmittel für ein solches Waschsystem ist wie folgt zusammengesetzt (alle Angaben in Gewichts-Prozent): 7% Alkylbenzolsulfonsäure, 9% anionische Tenside, 4% C12-C18 Na-Salze von Fettsäuren, 7% nicht-ionische Tenside, 0,7% Phosphonate, 3,2% Zitronensäure, 3,0% NaOH, 0,04% Antischaum, 5,7% 1,2-Propandiol, 0,1% Konservierungsstoffe, 2% Ethanol, 0,2% Farbstoff-Transfer-Inhibitor, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Im Rahmen der Erfindung erfolgt die Bestimmung die Reinigungsleistung bei 40°C unter Verwendung eines flüssigen Waschmittels wie vorstehend angegeben, wobei der Waschvorgang vorzugsweise für 30 Minuten erfolgt.

Der Weißheitsgrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Lipase wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann.

Die Lipaseaktivität wird in fachüblicher Weise bestimmt, und zwar vorzugsweise wie beschrieben in Bruno Stellmach, "Bestimmungsmethoden Enzyme für Pharmazie, Lebensmittelchemie, Technik, Biochemie, Biologie, Medizin" (Steinkopff Verlag Darmstadt, 1988, S. 172ff). Hierbei werden Lipase-haltige Proben zu einer Olivenölemulsion in emulgatorhaltigem Wasser gegeben und bei 30°C und pH 9,0 inkubiert. Dabei werden Fettsäuren freigesetzt. Diese werden mit einem Autotitrator über 20min. laufend mit 0,01 N Natronlauge titriert, so dass der pH-Wert konstant bleibt ("pH-stat-Titration"). Anhand des Natronlauge-Verbrauchs erfolgt mittels Bezug auf eine Referenzlipaseprobe die Bestimmung der Lipaseaktivität.

Ein alternativer Test zur Feststellung der lipolytischen Aktivität der erfindungsgemäßen Lipasen ist ein optisches Messverfahren, bevorzugt ein photometrisches Verfahren. Der hierfür geeignete Test umfasst die Lipase-abhängige Spaltung des Substrats para-Nitrophenol-butyrate (pNP-butyrate). Dieses wird durch die Lipase in para-Nitrophenolat und Butyrat gespalten. Die Anwesenheit von para-Nitrophenolat kann unter Verwendung eines Photometers, z.B. des Tecan Sunrise Geräts und der XFLUOR Software, bei 405 nm ermittelt werden und ermöglicht somit einen Rückschluss auf die enzymatische Aktivität der Lipase.

In vielfaltigen Ausführungsformen weist die erfindungsgemäße Lipase eine signifikant verminderte Geruchsemission gegenüber einer Lipase gemäß SEQ ID NO:1 und/oder einer Lipase gemäß SEQ ID NO:2 auf. "Signifikant" bedeutet in diesem Zusammenhang, dass die erfindungsgemäße Lipase höchstens 10%, 20%, 30%, 40%, 50%, 60%, 70%, 75%, 80%, 85%, 90%, 95% oder 98% der Geruchsintensität aufweist, die für eine Lipase gemäß SEQ ID NO:1 und/oder einer Lipase gemäß SEQ ID NO:2 bestimmt wird. In einem beispielhaften Verfahren zur Bestimmung der Geruchsintensität, die durch Lipasen hervorgerufen wird, werden Tücher (diese können bei Bedarf aus verschiedenen Materialien wie Baumwolle, Polyester, Polyamiden, Viscose, Meryl und Elasthan umfassen) mit Textilien unter den oben genannten Bedingungen gewaschen. Die Tücher werden anschließend solange luftdicht in Probenflaschen aufbewahrt bis ihre Geruchsintensität durch eine Prüfergruppe - das so genannte Sensorik-Panel - bestimmt wird.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. M. Bender et al., J. Am. Chem. Soc. 88, 24 (1966), S. 5890-5913) erfolgen.

Proteine können über die Reaktion mit einem Antiserum oder einem bestimmten Antikörper zu Gruppen immunologisch verwandter Proteine zusammengefasst werden. Die Angehörigen einer solchen Gruppe zeichnen sich dadurch aus, dass sie dieselbe, von einem Antikörper erkannte antigene Determinante aufweisen. Sie sind daher einander strukturell so ähnlich, dass sie von einem Antiserum oder bestimmten Antikörpern erkannt werden. Einen weiteren Erfindungsgegenstand bilden daher Lipasen, die dadurch gekennzeichnet sind, dass sie mindestens eine und zunehmend bevorzugt zwei, drei oder vier übereinstimmende antigene Determinanten mit einer erfindungsgemäßen Lipase aufweisen. Solche Lipasen sind auf Grund ihrer immunologischen Übereinstimmungen den erfindungsgemäßen Lipasen strukturell so ähnlich, dass auch von einer gleichartigen Funktion auszugehen ist.

Zusätzlich zu den vorstehend erläuterten Aminosäureveränderungen können erfindungsgemäße Lipasen weitere Aminosäureveränderungen, insbesondere Aminosäure-Substitutionen, -Insertionen oder-Deletionen, aufweisen. Solche Lipasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Lipasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Lipase noch weiter erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Lipase, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein.

Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt P208N die Substitution von Prolin an Position 95 durch Asparagin, P208PA die Insertion von Alanin nach der Aminosäure Prolin an Position 208 und P208* oder ΔP208 die Deletion von Prolin an Position 208. Diese Nomenklatur ist dem Fachmann auf dem Gebiet der Enzymtechnologie bekannt.

Ein weiterer Gegenstand der Erfindung ist daher eine Lipase, die dadurch gekennzeichnet ist, dass sie aus einer Lipase wie vorstehend beschrieben als Ausgangsmolekül erhältlich ist durch ein- oder mehrfache konservative Aminosäuresubstitution, wobei die Lipase in der Zählung gemäß SEQ ID NO. 1 noch mindestens eine der erfindungsgemäßen Aminosäuresubstitutionen an den Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, vorzugsweise 208, 211 oder 253, in SEQ ID NO:1 entsprechen, aufweist, wie vorstehend beschrieben. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Lipase dadurch gekennzeichnet, dass sie aus einer erfindungsgemäßen Lipase als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die im Ausgangsmolekül enthaltene mindestens eine mutierte Aminosäurereste an den Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, vorzugsweise 208, 211 oder 253, in SEQ ID NO:1 entsprechen, noch vorhanden ist.

So ist es beispielsweise möglich, an den Termini oder in den Loops des Enzyms einzelne Aminosäuren zu deletieren, ohne dass dadurch die lipolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre lipolytische Aktivität, d.h. ihre lipolytische Aktivität entspricht mindestens derjenigen des Ausgangsenzyms, d.h. in einer bevorzugten Ausführungsform beträgt die lipolytische Aktivität mindestens 80, vorzugsweise mindestens 90 % der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Alternativ oder ergänzend ist die Lipase dadurch gekennzeichnet, dass sie aus einer erfindungsgemäßen Lipase als Ausgangsmolekül erhältlich ist durch ein- oder mehrfache konservative Aminosäuresubstitution, wobei die Lipase mindestens eine der Aminosäuresubstitutionen an den Positionen, die den Positionen 83, 86, 87, 89, 90, 92, 93, 95, 113, 174, 202, 203, 205, 206, 207, 208, 209, 211, 227, 252, 253, 256, 258, 259, 260, 264, oder 267, gemäß SEQ ID NO:1 entsprechen, aufweist, vorzugsweise mindestens eine der Substitutionen P208N, F211N oder P253N.

In weiteren Ausführungsformen ist die Lipase dadurch gekennzeichnet, dass sie aus einer erfindungsgemäßen Lipase als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Lipase mindestens eine der Aminosäuresubstitutionen an den Positionen, die den Positionen 83, 86, 87, 89, 90, 92, 93, 95, 113, 174, 202, 203, 205, 206, 207, 208, 209, 211, 227, 252, 253, 256, 258, 259, 260, 264, oder 267, gemäß SEQ ID NO:1 entsprechen, umfasst, wobei die mindestens eine Aminosäuresubstitution aus der aus 208N und 253N bestehenden Gruppe ausgewählt ist.

Die weiteren Aminosäurepositionen werden hierbei durch ein Alignment der Aminosäuresequenz einer erfindungsgemäßen Lipase mit der Aminosäuresequenz der Lipase aus *Thermomyces lanuginosus,* wie sie in SEQ ID NO:1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Lipase eine höhere Zahl von Aminosäurenresten umfasst als die Lipase aus *Thermomyces lanuginosus* gemäß SEQ ID NO:1. Ausgehend von den genannten Positionen in der Aminosäuresequenz der Lipase aus *Thermomyces lanuginosus* sind die Veränderungspositionen in einer erfindungsgemäßen Lipase diejenigen, die eben diesen Positionen in einem Alignment zugeordnet sind.

Vorteilhafte Positionen für Sequenzveränderungen, insbesondere Substitutionen, der Lipase aus *Thermomyces lanuginosus,* die übertragen auf homologe Positionen der erfindungsgemäßen Lipasen bevorzugt von Bedeutung sind und der Lipase vorteilhafte funktionelle Eigenschaften verleihen, sind demnach die Positionen 83, 86, 87, 89, 90, 92, 93, 95, 113, 174, 202, 203, 205, 206, 207, 208, 209, 211, 227, 252, 253, 256, 258, 259, 260, 264, oder 267 zuzuordnen in einem Alignment mit SEQ ID NO:1 und damit in der Zählung gemäß SEQ ID NO:1. In den genannten Positionen liegen in dem Wildtypmolekül der Lipase aus *Thermomyces lanuginosus* folgende Aminosäurereste: S83, I86, E87, W89, I90, N92, L93, F95, F113, P174, I202, V203, R205, L206, P207, P208, R209, F211, L227, I252, P253, P256, H258, L259, W260, L264 und T267.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Lipasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Lipase aus *Thermomyces lanuginosus* gemäß SEQ ID NO:1 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Lipase-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Lipase anwendbar. Demnach umfasst ein erfindungsgemäßes Verfahren ferner einen oder mehrere der folgenden Verfahrensschritte:
a) Einbringen einer ein- oder mehrfachen konservativen Aminosäuresubstitution, wobei die Lipase mindestens eine Aminosäuresubstitution an den Positionen, die den Positionen 83, 86, 87, 89, 90, 92, 93, 95, 113, 174, 202, 203, 205, 206, 207, 208, 209, 211, 227, 252, 253, 256, 258, 259, 260, 264 und 267 gemäß SEQ ID NO:1 entsprechen, umfasst, wobei die mindestens eine Aminosäuresubstitution aus der aus 208N und 253N bestehenden Gruppe ausgewählt ist;
b) Veränderung der Aminosäuresequenz durch Fragmentierung, Deletions-, Insertions- oder Substitutionsmutagenese derart, dass die Lipase eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Lipase mindestens eine Aminosäuresubstitution an den Positionen, die den Positionen 83, 86, 87, 89, 90, 92, 93, 95, 113, 174, 202, 203, 205, 206, 207, 208, 209, 211, 227, 252, 253, 256, 258, 259, 260, 264 und 267 gemäß SEQ ID NO:1 entsprechen, umfasst, wobei die mindestens eine Aminosäuresubstitution aus der aus 208N und 253N bestehenden Gruppe ausgewählt ist.

In verschiedenen Ausführungsformen des Herstellungsverfahrens umfasst die erfindungsgemäße Lipase ferner die Aminosäuresubstitutionen T231R und/oder N233R, jeweils bezogen auf die Nummerierung gemäß SEQ ID NO:1. Diese Substitutionen sind vorzugsweise beide vorhanden und mit mindestens einer der hierin offenbarten Substitutionen kombiniert.

Sämtliche oben im Zusammenhang mit den Lipasen gemachten Ausführungen gelten auch für die erfindungsgemäßen Verfahren.

In weiteren Ausgestaltungen der Erfindung ist die erfindungsgemäße Lipase beziehungsweise die mit einem erfindungsgemäßen Verfahren hergestellte Lipase noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, oder 98,8% identisch zu der in SEQ ID NO:1 angegebenen Aminosäuresequenz über deren Gesamtlänge. Alternativ ist die erfindungsgemäße Lipase beziehungsweise die mit einem erfindungsgemäßen Verfahren hergestellte Lipase noch mindestens zu 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, oder 98% identisch zu einer der in SEQ ID Nos:2-5 angegebenen Aminosäuresequenzen über deren Gesamtlänge. Die Lipase beziehungsweise die mit einem erfindungsgemäßen Verfahren hergestellte Lipase weist mindestens eine Aminosäuresubstitution an den Positionen 83, 86, 87, 89, 90, 92, 93, 95, 113, 174, 202, 203, 205, 206, 207, 208, 209, 211, 227, 252, 253, 256, 258, 259, 260, 264 oder 267, jeweils bezogen auf die Nummerierung gemäß SEQ ID NO:1, auf, wobei die mindestens eine Aminosäuresubstitution aus der Gruppe bestehend aus 208N und 253N, jeweils bezogen auf die Nummerierung gemäß SEQ ID NO:1, ausgewählt ist. In erfindungsgemässen

Ausführungsformen umfasst die Lipase zwei der Aminosäuresubstitutionen P208N, und P253N. Ein weiterer Gegenstand der Erfindung ist eine zuvor beschriebene Lipase, die zusätzlich stabilisiert ist, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt. Möglichkeiten der Stabilisierung sind beispielsweise:
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Lipase wie vorstehend beschrieben, die dadurch gekennzeichnet ist, dass sie mindestens eine chemische Modifikation aufweist. Eine Lipase mit einer solchen Veränderung wird als Derivat bezeichnet, d.h. die Lipase ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Lipasen mit spezifischen Inhibitoren ist diesbezüglich möglich.

Betreffend alle vorstehend beschriebenen Lipasen beziehungsweise Lipasevarianten und/oder Derivate sind im Rahmen der vorliegenden Erfindung diejenigen besonders bevorzugt, deren Geruchsemission mindestens derjenigen der Lipasen gemäß SEQ ID NO:3-5 entspricht, wobei die Intensität der Geruchsemission in einem Verfahren bestimmt wird wie vorstehend beschrieben.

Ein weiterer Gegenstand der Erfindung ist eine Nukleinsäure, die für eine erfindungsgemäße Lipase codiert, sowie ein Vektor enthaltend eine solche Nukleinsäure, insbesondere ein Klonierungsvektor oder ein Expressionsvektor.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Lipasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Lipase beinhaltet, insbesondere eine, die die Lipase in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Lipasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Lipase funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung Bacillus, anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebakterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.*

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Lipasen herzustellen. Ein weiterer Gegenstand der Erfindung ist daher ein Verfahren zur Herstellung einer Lipase umfassend
a) Kultivieren einer erfindungsgemäßen Wirtszelle, und
b) Isolieren der Lipase aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Lipasen. Alle Fermentationsverfahren, die auf einem entsprechenden Verfahren zur Herstellung einer erfindungsgemäßen Lipase beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Lipase erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Lipase kann aus dem Fermentationsmedium geerntet werden. Ein solches Fermentationsverfahren ist gegenüber einer Isolation der Lipase aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Lipase in das Fermentationsmedium sezernieren. Ohne Sekretion kann alternativ die Isolation der Lipase aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Lipasen herzustellen.

Ein weiterer Gegenstand der Erfindung ist ein Mittel, das dadurch gekennzeichnet ist, dass es eine erfindungsgemäße Lipase wie vorstehend beschrieben enthält. Bevorzugt ist das Mittel als ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Lipase alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Lipase mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Lipase mit einem Tensid und/oder einem Builder (Gerüststoff) und/oder einer Persauerstoffverbindung und/oder einem Bleichaktivator kann ein solcher Synergismus erreicht werden.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

In weiteren Ausführungsformen der Erfindung ist das Mittel dadurch gekennzeichnet, dass es
(a) 1-85 Gew.-%, vorzugsweise 5-65 Gew.-%, Tenside enthält; und/oder
(a) 0-45 Gew.-%, vorzugsweise 0,1-15 Gew.-%, Builder (Gerüststoffe) enthält; und/oder
(a) 0,0005-15 Gew.-%, vorzugsweise 0,001-5 Gew.-%, Protease enthält; und/oder
(a) 0,0005-15 Gew.-%, vorzugsweise 0,001-5 Gew.-%, Amylase enthält; und/oder
(a) 0,00005-15 Gew.-%, vorzugsweise 0,0001-5 Gew.-%, Mannanase enthält; und/oder
(a) 0,00005-15 Gew.-%, vorzugsweise 0,0001-5 Gew.-%, Cellulase/Pektatlyase enthält; und/oder
(a) 0,00005-15 g/Waschladung, vorzugsweise 0,0001-5 g/Waschladung, Xanthanlyase enthält; und/oder
(a) 0,00005-15 g/Waschladung, vorzugsweise 0,00005-15 g/Waschladung, Endoglucanase, die fähig ist Xanthan zu verdauen, enthält.

Ein erfindungsgemäßes Mittel enthält die Lipase vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. Darüber hinaus kann das erfindungsgemäße Mittel die Lipase vorteilhafterweise in einer Menge von 0,00005-15 Gew.-% bezogen auf das aktive Enzym, vorzugsweise von 0,0001-5 Gew.-% und besonders bevorzugt von 0,001-1 Gew.-% enthalten. Ferner kann die in dem Mittel enthaltene Lipase, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Lipase mit einer bei Raumtemperatur oder bei Abwesenheit von Wasser für die Lipase undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) in gelförmiger oder dosierbeutelförmiger (Pouches) Form vorliegt, und/oder
(d) als Einkomponentensystem vorliegt, oder
(e) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine Lipase enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Lipasen unterscheidbare - Lipasen, sowie deren Gemische. Weitere Enzyme sind in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Lipase und einem weiteren Enzym eines erfindungsgemäßen Mittels, darunter insbesondere zwischen der genannten Lipase und einer Amylase und/oder einer Protease und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Lipase katalytisch aktiv wird, insbesondere derart, dass die Lipase in einer Menge von 40µg bis 4g, vorzugsweise von 50µg bis 3g, besonders bevorzugt von 100µg bis 2g und ganz besonders bevorzugt von 200µg bis 1g eingesetzt wird.

In verschieden Ausführungsformen zeichnet sich das oben beschriebene Verfahren dadurch aus, dass die Lipase bei einer Temperatur von 0-100°C, bevorzugt 0-60°C, weiter bevorzugt 20-45°C und am meisten bevorzugt bei 40°C eingesetzt wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Lipase bereichert werden und stellen dann Ausführungsformen der vorliegenden Erfindung dar. Alle Sachverhalte, Gegenstände und Ausführungsformen, die für erfindungsgemäße Lipasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Lipasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass gewünschtenfalls als einzige reinigungsaktive Komponente eine erfindungsgemäße Lipase mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Lipase aktiv wird. Hierunter sind Verfahren für Textilrohstoffe, Fasern oder Textilien mit natürlichen Bestandteilen bevorzugt.

Alle Sachverhalte, Gegenstände und Ausführungsformen, die für erfindungsgemäße Lipase und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

In einem weiteren Aspekt bezieht sich die vorliegende Erfindung auf die Verwendung einer erfindungsgemäßen Lipase oder einer nach einem erfindungsgemäßen Verfahren erhältliche Lipase in einem Wasch- oder Reinigungsmittel zur Entfernung von lipidhaltigen Anschmutzungen.

Die hierin beschriebenen Lipasen zeichnen sich insbesondere durch eine geringere Geruchsbildung beim Einsatz in Waschmitteln für Textilien aus. Eine entsprechende Verwendung zur Reduzierung von unerwünschter Geruchsbildung auf mit Lipase-haltigen Waschmitteln gewaschenen Textilien ist daher ebenfalls Bestandteil der Erfindung. Die Erfindung betrifft daher insbesondere auch die Verwendung einer Lipase wie hierin beschrieben in einem Wasch- oder Reinigungsmittel zur Verminderung von Schlechtgerüchen von damit gewaschenen Textilien.

Alle Sachverhalte, Gegenstände und Ausführungsformen, die für erfindungsgemäße Lipase und sie enthaltende Mittel beschrieben sind, sind auch auf diese Erfindungsgegenstände anwendbar.

### Beispiele

### Beispiel 1: Waschbedingungen und Geruchstest

### Übersicht über die Mutationen:

| **Variante** | **Sequenz** | | | **SEQ ID NO:** |
|---|---|---|---|---|
| Variante 1 (Vergleichs beispiel) | | | | 1 |
| Variante 2 (erfindungsgemäß) | P208N | T231R | N233R | 3 |
| Variante 2 (erfindungsgemäß) | F211N | T231R | N233R | 4 |
| Variante 3 (erfindungsgemäß) | P253N | T231R | N233R | 5 |

Flüssigwaschmittelformulierungen (siehe Tabelle 1):
Eine Formulierung enthielt die Referenzlipase mit der Aminosäuresequenz gemäß SEQ ID NO:1,
weitere Formulierungen die erfindungsgemäßen Lipasen mit der Sequenz gemäß SEQ ID NO:3-5.

Für den Geruchstest wurde eine Mikrotiterplatte mit sauberen Textilstreifen (Baumwolle, 10mm) vorbereitet. Die Waschmittellösung und die Mikrotiterplatte wurden auf 40°C vorgeheizt. Die Textilstreifen wurden für 1 h bei 40°C mit einer Lipase-haltigen Waschlauge gewaschen. Das Waschmittel wurde dazu auf eine Konzentration von 4,7g/L in der Waschlauge verdünnt. Die Lipasekonzentration betrug 0,4%. Als Referenz wurde die Waschmatrix ohne Lipase eingesetzt.

Nach dem Waschen wurden die Textilstreifen getrocknet und dann mit Fettanschmutzungen verschmutzt. Die zugegebenen Fette wurden im folgenden Verhältnis zugegeben:
Rama (Margarine) : Butter: Hautfett: Olivenöl : Mineralöl im Verhältnis 1 : 1 : 1 : 2 : 2.

Danach wurden die mit Fettanschmutzungen präparierten Textilstreifen in luftdichten Probenbehältern separat für 7 Tage bei Raumtemperatur verwahrt. Für den Geruchstest wurde eine Prüfergruppe von 6 Personen (Sensorik-Panel) eingesetzt, um nach 7 Tagen die Textilstreifen abzuriechen und deren Geruchsintensität zu bewerten. Wenn Lipase auf den Textilien vorhanden ist, werden die fettigen Anschmutzungen abgebaut und erzeugen einen Schlechtgeruch. Der Geruchstest umfasst die Bewertung auf einer Skala von 1 bis 5, wobei geringere Werte im Hinblick auf den Geruch vorteilhaft sind. Die Ergebnisse sind in Tabelle 2 dargestellt.

**Tabelle 1: Verwendete Waschmittelformulierungen**

| Inhaltsstoff | V1 | E1 |
|---|---|---|
| LAS-Na | 10,6 | 10,6 |
| FA 7 EO | 3 | 3 |
| C16-18 Na-Seife | 1,2 | 1,2 |
| HEDP-Na₄ | 0,81 | 0,81 |
| Polyacrylat | 2,2 | 2,2 |
| Natriumsilkat 2.1 | 5,7 | 5,7 |
| Natriumcarbonat | 22 | 22 |
| Natriumpercarbonat | 10 | 10 |
| TAED | 3 | 3 |
| Acylhydrazon | - | 0,09 |
| Carboxymethylcellulose | 2,5 | 2,5 |
| Entschäumer | 0,046 | 0,046 |
| Protease/Amylase/Cellulase | 0,61 | 0,61 |
| Lipase | 0,005 | 0,005 |
| Optischer Aufheller | 0,183 | 0,183 |
| Parfum | 0,3 | 0,3 |
| Natriumsulfat | auf 100 | auf 100 |

**Tabelle 2: Ergebnisse des Geruchstests**

| Probe | Baumwolle |
|---|---|
| Referenz (Waschmittelmatrix ohne Lipase) | 0,5 |
| Waschmittelmatrix + Referenzlipase (SEQ ID NO:2) | 1,8 |
| Waschmittelmatrix + erfindungsgemäße Lipase (SEQ ID NO:3) | 1,1 |
| Waschmittelmatrix + erfindungsgemäße Lipase (SEQ ID NO:4) | 0,9 |
| Waschmittelmatrix + erfindungsgemäße Lipase (SEQ ID NO:5) | 1,3 |

Die Ergebnisse zeigen, dass sich die erfindungsgemäßen Lipasevarianten gegenüber dem Wildtypenzym durch eine verminderte Bildung von Schlechtgerüchen auszeichnen.

## Patentansprüche

1. Lipase umfassend eine Aminosäuresequenz, die mindestens 70 % Sequenzidentität mit der in SEQ ID NO:1 angegebenen Aminosäuresequenz über deren Gesamtlänge aufweist und die mindestens eine Aminosäuresubstitution an mindestens einer der Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, jeweils bezogen auf die Nummerierung gemäß SEQ ID NO:1, entsprechen, aufweist, wobei die mindestens eine Aminosäuresubstitution aus der Gruppe bestehend aus 208N und 253N, jeweils bezogen auf die Nummerierung gemäß SEQ ID NO:1, ausgewählt ist.

2. Lipase nach Anspruch 1, wobei die Lipase die Aminosäuresubstitution(en) P208N, und/oder P253N, jeweils bezogen auf die Nummerierung gemäß SEQ ID NO:1, umfasst.

3. Lipase, **dadurch gekennzeichnet, dass**
(a) sie aus einer Lipase nach Anspruch 1-2 als Ausgangsmolekül erhältlich ist durch ein- oder mehrfache konservative Aminosäuresubstitution, wobei die Lipase mindestens eine Aminosäuresubstitution an mindestens einer der Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, vorzugsweise 208, 211 oder 253, jeweils bezogen auf die Nummerierung gemäß SEQ ID NO:1, entsprechen, umfasst, wobei die mindestens eine Aminosäuresubstitution aus der Gruppe bestehend aus 208N und 253N, jeweils bezogen auf die Nummerierung gemäß SEQ ID NO:1, ausgewählt ist; und/oder
(b) sie aus einer Lipase nach Anspruch 1-2 als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Lipase mindestens eine Aminosäuresubstitution an mindestens einer der Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, vorzugsweise 208, 211 oder 253,jeweils bezogen auf die Nummerierung gemäß SEQ ID NO:1, entsprechen, umfasst, wobei die mindestens eine Aminosäuresubstitution aus der Gruppe bestehend aus 208N und 253N, jeweils bezogen auf die Nummerierung gemäß SEQ ID NO:1, ausgewählt ist.

4. Lipase nach einen der Ansprüche 1-3, wobei die Lipase ferner die Aminosäuresubstitutionen T231R und N233R, jeweils bezogen auf die Nummerierung gemäß SEQ ID NO:1, umfasst.

5. Verfahren zur Herstellung einer Lipase umfassend das Substituieren mindestens einer Aminosäure an mindestens einer der Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, vorzugsweise 208, 211 oder 253, jeweils bezogen auf die Nummerierung gemäß SEQ ID NO:1, entsprechen, in einer Ausgangslipase, die mindestens 70 % Sequenzidentität zu der in SEQ ID NO:1 angegebenen Aminosäuresequenz über deren Gesamtlänge aufweist, derart, dass die Lipase an mindestens einer der Positionen eine Aminosäuresubstitution aufweist, ausgewählt aus P208N und P253N.

6. Verfahren nach Anspruch 5, ferner umfassend einen oder beide der folgenden Verfahrensschritte:
(a) Substituieren mindestens einer Aminosäure durch ein- oder mehrfache konservative Aminosäuresubstitution, wobei die Lipase mindestens eine Aminosäuresubstitution an mindestens einer der Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, jeweils bezogen auf die Nummerierung gemäß SEQ ID NO:1, entsprechen, umfasst, wobei die mindestens eine Aminosäuresubstitution aus der Gruppe bestehend aus 208N und 253N, jeweils bezogen auf die Nummerierung gemäß SEQ ID NO:1, ausgewählt ist;
b) Veränderung der Aminosäuresequenz durch Fragmentierung, Deletions-, Insertions- oder Substitutionsmutagenese derart, dass die Lipase eine Aminosäuresequenz umfasst, die über eine Länge von mindestens 190, 200, 210, 220, 230, 240, 250, 260 oder 269 zusammenhängenden Aminosäuren mit dem Ausgangsmolekül übereinstimmt, wobei die Lipase mindestens eine Aminosäuresubstitution an mindestens einer der Positionen, die Positionen 83, 86, 87, 89, 90, 92, 93, 95, 113, 174, 202, 203, 205, 206, 207, 208, 209, 211, 227, 252, 253, 256, 258, 259, 260, 264, oder 267, jeweils bezogen auf die Nummerierung gemäß SEQ ID NO:1, entsprechen, umfasst, wobei die mindestens eine Aminosäuresubstitution aus der Gruppe bestehend aus 208N und 253N, jeweils bezogen auf die Nummerierung gemäß SEQ ID NO:1, ausgewählt ist.

7. Verfahren nach Anspruch 5 oder 6, wobei die Lipase ferner die Aminosäuresubstitutionen T231R und N233R, jeweils bezogen auf die Nummerierung gemäß SEQ ID NO:1, umfasst.

8. Nukleinsäure codierend für eine Lipase nach einem der Ansprüche 1-4 oder codierend für eine nach einem Verfahren der Ansprüche 5-7 erhaltene Lipase.

9. Vektor enthaltend eine Nukleinsäure nach Anspruch 8, insbesondere ein Klonierungsvektor oder ein Expressionsvektor.

10. Nicht menschliche Wirtszelle, die eine Nukleinsäure nach Anspruch 8 oder einen Vektor nach Anspruch 9 beinhaltet, oder die eine Lipase nach einem der Ansprüche 1-4 beinhaltet, oder die eine nach einem Verfahren der Ansprüche 5-7 erhaltene Lipase beinhaltet, insbesondere eine, die die Lipase in das die Wirtszelle umgebende Medium sezerniert.

11. Verfahren zur Herstellung einer Lipase umfassend
a) Kultivieren einer Wirtszelle gemäß Anspruch 10
b) Isolieren der Lipase aus dem Kulturmedium oder aus der Wirtszelle.

12. Mittel, insbesondere ein Wasch- oder Reinigungsmittel, **dadurch gekennzeichnet, dass** es mindestens eine Lipase nach einem der Ansprüche 1-4 oder eine nach einem Verfahren der Ansprüche 5-7 erhaltene Lipase enthält,
a) insbesondere ist die Lipase in einer Menge bis zu 1 Gew.-% bezogen auf aktives Protein enthalten; und/oder
b) wobei insbesondere das Mittel ein flüssiges Mittel ist.

13. Verfahren zur Reinigung von Textilien oder harten Oberflächen, **dadurch gekennzeichnet, dass** in mindestens einem Verfahrensschritt ein Mittel nach Anspruch 12 angewendet wird, oder dass in mindestens einem Verfahrensschritt eine Lipase nach einem der Ansprüche 1-4 oder eine nach einem Verfahren der Ansprüche 5-7 erhaltene Lipase katalytisch aktiv wird.

14. Verwendung einer Lipase nach einem der Ansprüche 1-4 oder einer nach einem Verfahren der Ansprüche 5-7 erhältlichen Lipase in einem Wasch- oder Reinigungsmittel zur Entfernung von lipidhaltigen Anschmutzungen oder zur Verminderung von Schlechtgerüchen von damit gewaschenen Textilien.

## Claims

1. A lipase comprising an amino acid sequence which has at least 70% sequence identity with the amino acid sequence given in SEQ ID NO:1 over the entire length thereof and which has at least one amino acid substitution at at least one of the positions corresponding to positions 83, 86, 87, 89, 90, 92, 93, 95, 113, 174, 202, 203, 205, 206, 207, 208, 209, 211, 227, 252, 253, 256, 258, 259, 260, 264, or 267, in each case based on the numbering according to SEQ ID NO:1, wherein the at least one amino acid substitution is selected from the group consisting of 208N and 253N, in each case based on the numbering according to SEQ ID NO:1.

2. The lipase according to claim 1, wherein the lipase comprises the amino acid substitution(s) P208N and/or P253N, in each case based on the numbering according to SEQ ID NO:1.

3. A lipase, **characterized in that**
(a) said lipase can be obtained from a lipase according to claim 1-2 as a starting molecule by one or more conservative amino acid substitutions, the lipase comprising at least one amino acid substitution at at least one of the positions corresponding to positions 83, 86, 87, 89, 90, 92, 93, 95, 113, 174, 202, 203, 205, 206, 207, 208, 209, 211, 227, 252, 253, 256, 258, 259, 260, 264, or 267, preferably 208, 211 or 253, in each case based on the numbering according to SEQ ID NO:1, the at least one amino acid substitution being selected from the group consisting of 208N and 253N, in each case based on the numbering according to SEQ ID NO: 1; and/or
(b) said lipase can be obtained from a lipase according to claim 1-2 as a starting molecule by fragmentation or deletion, insertion or substitution mutagenesis and comprises an amino acid sequence which matches that of the starting molecule over a length of at least 190, 200, 210, 220, 230, 240, 250, 260 or 269 contiguous amino acids, the lipase having at least one amino acid substitution at at least one of the positions corresponding to positions 83, 86, 87, 89, 90, 92, 93, 95, 113, 174, 202, 203, 205, 206, 207, 208, 209, 211, 227, 252, 253, 256, 258, 259, 260, 264, or 267, preferably 208, 211 or 253, in each case based on the numbering according to SEQ ID NO:1, the at least one amino acid substitution being selected from the group consisting of 208N and 253N, in each case based on the numbering according to SEQ ID NO:1.

4. The lipase according to one of claims 1 to 3, wherein the lipase further comprises the amino acid substitutions T231R and N233R, in each case based on the numbering according to SEQ ID NO:1.

5. A method for producing a lipase, comprising the substitution of at least one amino acid at at least one of the positions corresponding to positions 83, 86, 87, 89, 90, 92, 93, 95, 113, 174, 202, 203, 205, 206, 207, 208, 209, 211, 227, 252, 253, 256, 258, 259, 260, 264, or 267, preferably 208, 211 or 253, in each case based on the numbering according to SEQ ID NO:1, in a starting lipase which has at least 70% sequence identity with the amino acid sequence given in SEQ ID NO:1 over the entire length thereof, such that the lipase has an amino acid substitution selected from P208N and P253N at at least one of the positions.

6. The method according to claim 5, further comprising one or both of the following method steps:
a) substituting at least one amino acid by one or more conservative amino acid substitutions, wherein the lipase has at least one amino acid substitution at at least one of the positions corresponding to positions 83, 86, 87, 89, 90, 92, 93, 95, 113, 174, 202, 203, 205, 206, 207, 208, 209, 211, 227, 252, 253, 256, 258, 259, 260, 264, or 267, in each case based on the numbering according to SEQ ID NO:1, wherein the at least an amino acid substitution is selected from the group consisting of 208N and 253N, in each case based on the numbering according to SEQ ID NO:1;
b) altering the amino acid sequence by fragmentation or deletion, insertion or substitution mutagenesis in such a way that the lipase comprises an amino acid sequence which matches that of the starting molecule over a length of at least 190, 200, 210, 220, 230, 240, 250, 260 or 269 contiguous amino acids, wherein the lipase comprises at least one amino acid substitution at at least one of the positions corresponding to positions 83, 86, 87, 89, 90, 92, 93, 95, 113, 174, 202, 203, 205, 206, 207, 208, 209, 211, 227, 252, 253, 256, 258, 259, 260, 264, or 267, in each case based on the numbering according to SEQ ID NO:1, wherein the at least one amino acid substitution is selected from the group consisting of 208N and 253N, in each case based on the numbering according to SEQ ID NO:1.

7. The method according to claim 5 or 6, wherein the lipase further comprises the amino acid substitutions T231R and N233R, in each case based on the numbering according to SEQ ID NO:1.

8. A nucleic acid coding for a lipase according to one of claims 1 to 4 or coding for a lipase obtained by a method of claims 5 to 7.

9. A vector containing a nucleic acid according to claim 8, in particular a cloning vector or an expression vector.

10. A non-human host cell that contains a nucleic acid according to claim 8 or a vector according to claim 9, or that contains a lipase according to one of claims 1 to 4, or that contains a lipase obtained by a method of claims 5 to 7, in particular a non-human host cell that secretes the lipase into the medium surrounding the host cell.

11. A method for producing a lipase, comprising
a) cultivating a host cell according to claim 10
b) isolating the lipase from the culture medium or from the host cell.

12. An agent, in particular a washing or cleaning agent, **characterized in that** it contains at least one lipase according to one of claims 1 to 4 or a lipase that can be obtained by a method of claims 5 to 7,
a) in particular, the lipase is contained in an amount of up to 1 wt.% based on active protein; and/or
b) the agent being in particular a liquid agent.

13. A method for cleaning textiles or hard surfaces, **characterized in that** an agent according to claim 12 is used in at least one method step, or **in that** a lipase according to one of claims 1 to 4 or a lipase that can be obtained by a method of claims 5 to 7 is catalytically activated in at least one method step.

14. The use of a lipase according to one of claims 1 to 4 or of a lipase obtainable by a method of claims 5 to 7 in a washing or cleaning agent for removing lipid-containing stains or for reducing bad odors from textiles washed using said agent.

## Revendications

1. Lipase comprenant une séquence d'acides aminés, laquelle présente au moins 70 % d'identité de séquence avec la séquence d'acides aminés donnée dans la SEQ ID NO : 1 sur toute sa longueur et laquelle présente au moins une substitution d'acide aminé à au moins une des positions correspondant aux positions 83, 86, 87, 89, 90, 92, 93, 95, 113, 174, 202, 203, 205, 206, 207, 208, 209, 211, 227, 252, 253, 256, 258, 259, 260, 264 ou 267, respectivement rapporté à la numérotation selon la SEQ ID NO : 1, où l'au moins une substitution d'acide aminé est choisie dans le groupe constitué par 208N et 253N, respectivement rapporté à la numérotation selon la SEQ ID NO : 1.

2. Lipase selon la revendication 1, où la lipase comprend la ou les substitutions d'acides aminés P208N et/ou P253N, respectivement rapporté à la numérotation selon SEQ ID NO : 1.

3. Lipase, **caractérisée en ce**
(a) **qu'**elle peut être obtenue à partir d'une lipase selon les revendications 1 à 2 en tant que molécule de départ par au moins une substitution d'acide aminé conservatrice, où la lipase comprend au moins une substitution d'acide aminé à l'au moins une des positions correspondant aux positions 83, 86, 87, 89, 90, 92, 93, 95, 113, 174, 202, 203, 205, 206, 207, 208, 209, 211, 227, 252, 253, 256, 258, 259, 260, 264 ou 267, de préférence 208, 211 ou 253, respectivement rapporté à la numérotation selon la SEQ ID NO : 1, où l'au moins une substitution d'acide aminé est choisie dans le groupe constitué par 208N et 253N, respectivement rapporté à la numérotation selon la SEQ ID NO : 1 ; et ou
(b) **qu'**elle peut être obtenue à partir d'une lipase selon la revendication 1 à 2 en tant que molécule de départ par fragmentation, par mutagenèse par délétion, par mutagenèse par insertion ou par mutagenèse par substitution et comprend une séquence d'acides aminés, laquelle coïncide avec la molécule de départ sur une longueur d'au moins 190, 200, 210, 220, 230, 240, 250, 260 ou 269 acides aminés contigus, où la lipase comprend au moins une substitution d'acide aminé à l'au moins une des positions correspondant aux positions 83, 86, 87, 89, 90, 92, 93, 95, 113, 174, 202, 203, 205, 206, 207, 208, 209, 211, 227, 252, 253, 256, 258, 259, 260, 264 ou 267, de préférence 208, 211 ou 253, respectivement rapporté à la numérotation selon la SEQ ID NO : 1, où l'au moins une substitution d'acide aminé est choisie dans le groupe constitué par 208N et 253N, respectivement rapporté à la numérotation selon la SEQ ID NO : 1.

4. Lipase selon l'une quelconque des revendications 1 à 3, où la lipase comprend en outre les substitutions d'acides aminés T231R et N233R, respectivement rapporté à la numérotation selon la SEQ ID NO : 1.

5. Procédé de production d'une lipase comprenant la substitution d'au moins un acide aminé à au moins une des positions correspondant aux positions 83, 86, 87, 89, 90, 92, 93, 95, 113, 174, 202, 203, 205, 206, 207, 208, 209, 211, 227, 252, 253, 256, 258, 259, 260, 264 ou 267, de préférence 208, 211 ou 253, respectivement rapporté à la numérotation selon la SEQ ID NO : 1, dans une lipase de départ, laquelle présente au moins 70 % d'identité de séquence avec la séquence d'acides aminés donnée dans la SEQ ID NO : 1 sur toute sa longueur de telle sorte que la lipase présente une substitution d'acide aminé choisie entre P208N et P253N à l'au moins une des positions.

6. Procédé selon la revendication 5, comprenant en outre l'une ou les deux étapes suivantes :
a) la substitution d'au moins un acide aminé par au moins une substitution d'acide aminé conservatrice, où la lipase comprend au moins une substitution d'acide aminé à l'au moins une des positions correspondant aux positions 83, 86, 87, 89, 90, 92, 93, 95, 113, 174, 202, 203, 205, 206, 207, 208, 209, 211, 227, 252, 253, 256, 258, 259, 260, 264 ou 267, respectivement rapporté à la numérotation selon la SEQ ID NO : 1, où l'au moins une substitution d'acide aminé est choisie dans le groupe constitué par 208N et 253N, respectivement rapporté à la numérotation selon la SEQ ID NO : 1 ;
b) l'altération de la séquence d'acides aminés par fragmentation, par mutagenèse par délétion, par mutagenèse par insertion ou par mutagenèse par substitution de telle sorte que la lipase comprend une séquence d'acides aminés, laquelle coïncide avec la molécule de départ sur une longueur d'au moins 190, 200, 210, 220, 230, 240, 250, 260 ou 269 acides aminés contigus, où la lipase comprend au moins une substitution d'acide aminé à l'au moins une des positions correspondant aux positions 83, 86, 87, 89, 90, 92, 93, 95, 113, 174, 202, 203, 205, 206, 207, 208, 209, 211, 227, 252, 253, 256, 258, 259, 260, 264 ou 267, respectivement rapporté à la numérotation selon la SEQ ID NO : 1, où l'au moins une substitution d'acide aminé est choisie dans le groupe constitué par 208N et 253N, respectivement rapporté à la numérotation selon la SEQ ID NO : 1.

7. Procédé selon la revendication 5 ou 6, où la lipase comprend en outre les substitutions d'acides aminés T231R et N233R, respectivement rapporté à la numérotation selon la SEQ ID NO : 1.

8. Acide nucléique codant pour une lipase selon l'une quelconque des revendications 1 à 4 ou codant pour une lipase obtenue selon un procédé selon les revendications 5 à 7.

9. Vecteur contenant un acide nucléique selon la revendication 8, en particulier un vecteur de clonage ou un vecteur d'expression.

10. Cellule hôte non humaine, laquelle contient un acide nucléique selon la revendication 8 ou un vecteur selon la revendication 9, ou laquelle comprend une lipase selon l'une quelconque des revendications 1 à 4, ou laquelle contient une lipase obtenue selon un procédé selon les revendications 5 à 7, en particulier sécrétant la lipase dans le milieu environnant la cellule hôte.

11. Procédé de production d'une lipase comprenant
a) la culture d'une cellule hôte selon la revendication 10
b) l'isolement de la lipase à partir du milieu de culture ou de la cellule hôte.

12. Agent, en particulier agent de lavage ou de nettoyage, **caractérisé en ce qu'**il contient au moins une lipase selon l'une quelconque des revendications 1 à 4 ou une lipase obtenue selon un procédé selon les revendications 5 à 7,
a) en particulier, la lipase est contenue en une quantité allant jusqu'à 1 % en poids rapporté à la protéine active ; et/ou
b) où l'agent est en particulier un agent liquide.

13. Procédé de nettoyage des textiles ou des surfaces dures, **caractérisé en ce qu'**un agent selon la revendication 12 est utilisé dans au moins une étape, ou que, dans au moins une étape, une lipase selon l'une quelconque des revendications 1 à 4 ou une lipase obtenue selon un procédé selon les revendications 5 à 7 est catalytiquement active.

14. Utilisation d'une lipase selon l'une quelconque des revendications 1 à 4 ou d'une lipase obtenue selon un procédé selon les revendications 5 à 7 dans un agent de lavage ou de nettoyage destiné à l'élimination des salissures contenant des lipides ou destiné à la réduction des mauvaises odeurs des textiles lavés avec celui-ci.
